# FASCICULE DE BREVET EUROPEEN

(11) **EP 2 124 587 B1**
(45) Date de publication et mention de la délivrance du brevet: **22.10.2014**
(21) Numéro de dépôt: 08716948.8
(22) Date de dépôt: 19.02.2008
(51) Int. Cl.: A23C 9/137, A23L 1/10, A23L 1/308, A61K 31/716, A61P 3/04, A61P 3/10

(54) **PRODUIT ALIMENTAIRE SEMI-FLUIDE COMPRENANT DES FIBRES DE BETA-GLUCANE**
BETA-GLUCAN-FASERN ENTHALTENDES, HALBFLÜSSIGES NAHRUNGSMITTELPRODUKT
SEMI-FLUID FOOD PRODUCT COMPRISING BETA-GLUCAN FIBRES

(30) Priorité: 20.02.2007 FR 0753385
(43) Date de publication de la demande: 02.12.2009
(73) Titulaire: Compagnie Gervais Danone, 75009 Paris (FR)
(72) Inventeur: BOULAT, Céline, F-91530 Saint Chenon (FR); NOBLE, Olivier, F-91400 Orsay (FR); SCHMITT, Laurent, F-91430 Igny (FR); VAULOUP, Fabien, F-91300 Massy (FR)
(74) Mandataire: Regimbeau
(86) Numéro de dépôt international: PCT/EP2008/052001
(87) Numéro de publication internationale: WO 2008/101924

(56) Documents cités:
- EP-A2- 0 898 900
- WO-A-00/67592
- WO-A-02/082929
- WO-A-2006/040395
- US-A1- 2004 258 829
- US-A1- 2005 271 613

## Description

L'invention a pour but l'incorporation de fibres de β-glucane dans des produits laitiers.

Les fibres de β - glucane extraites de céréales sont reconnues pour leurs bénéfices santé. En particulier :
- elles permettent de réduire le taux de cholestérol sanguin, à partir d'une dose de 0,75 g de β - glucane,
- elles peuvent également permettre de diminuer le taux d'insuline post-prandial (réponse insulinémique) à des doses de l'ordre de 4 g, avec un bénéfice potentiel sur la gestion du poids.

Des extraits d'avoine ou d'orge enrichis en β - glucane, avec des taux de β - glucane compris entre 20% et 50%, sont disponibles commercialement. Ces produits restent cependant difficiles à utiliser dans des produits laitiers frais, notamment à cause des doses importantes à incorporer et des forts goûts de céréales générés.

Il est aussi fait référence à US 2004/258829 et WO 02/082929.

Un extrait purifié de β - glucane d'orge, avec une teneur en β - glucane supérieure à 70%, est proposé par la société Cargill sous le nom commercial de Barliv. Ce produit est beaucoup plus neutre en goût, mais il reste difficile à incorporer dans des produits laitiers fermentés.

Par ailleurs, l'ajout de fibres de β - glucane dans le mix laitier avant fermentation perturbe la formation du gel lactique, et on obtient, après fermentation, un caillé de mauvaise qualité et un déphasage important.

En outre, l'incorporation de quantités importantes de cet extrait dans une préparation aqueuse, une préparation de fruits par exemple, conduit à l'obtention d'une préparation très texturée, qui de plus gélifie au stockage au bout de quelques heures.

Le traitement thermique de la préparation aqueuse rajoute une difficulté à l'incorporation de cet ingrédient.

Des procédés permettant de faciliter l'incorporation de fibres texturantes (c'est-à-dire induisant une augmentation de viscosité du milieu dans lequel elles sont introduites) dans des produits laitiers sont déjà connus. Par exemple, la demande internationale WO 00/67592 enseigne l'ajout d'un dépresseur de viscosité

(maltodextrine, gomme guar hydrolysée, inuline) à une composition comprenant du glucomannane.

Cependant, outre un fort pouvoir texturant, les fibres de β - glucane possèdent également un fort pouvoir gélifiant. Or, le simple ajout d'un dépresseur de viscosité ne permet pas d'éviter la gélification (prise en masse) induite par les fibres de β - glucane.

Jusqu'à présent, pour fournir simultanément au consommateur un produit laitier et des fibres de β - glucane, des produits bi-compartiments étaient proposés : un compartiment contenant le produit laitier et un autre compartiment contenant des flocons riches en fibres de β - glucane à ajouter dans le produit laitier avant consommation. Outre le coût élevé de tels produits bi-compartiments, les résultats organoleptiques ne sont pas satisfaisants. En effet, les flocons sont soit trop durs si le produit mélangé est consommé immédiatement, soit pâteux si la consommation est plus tardive.

La préparation de produits laitiers fermentés contenant des doses significatives de β - glucane, et acceptables au niveau organoleptique, présente donc des difficultés non surmontées à ce jour.

D'une manière surprenante, les inventeurs ont démontré qu'il était possible de préparer une solution aqueuse thermisée semi-fluide contenant une quantité significative de fibres de β-glucane, par l'ajout d'un agent dépresseur de viscosité combiné à l'utilisation d'un procédé de refroidissement lent sous cisaillement.

Un premier objet de l'invention est donc une solution aqueuse thermisée semi-fluide comprenant des fibres de β - glucane, caractérisée en ce qu'elle comprend
a. 3% à 12% en poids, par rapport au poids total de la solution, desdites fibres de β - glucane, et
b. une quantité suffisante, comprise entre 5% et 30% en poids, par rapport au poids total de la solution, d'au moins un dépresseur de viscosité choisi dans le groupe constitué par les maltodextrines ayant un DE maximal de 18, la gomme de guar au moins partiellement hydrolysée, l'inuline et les fructo-oligosaccharides (FOS).

Dans le cadre de la présente invention, le dépresseur de viscosité choisi est de préférence de la gomme de guar au moins partiellement hydrolysée.

Par « thermisée », on entend « traité thermiquement pour éliminer les contaminants microbiologiques ». Ce traitement peut être une pasteurisation, une stérilisation, ou tout autre procédé thermique.

Par « semi-fluide », on entend une solution qui a une viscosité (mesurée à 10°C) inférieure à 10 000 mPa.s.

Les fibres de β - glucane sont des fibres alimentaires solubles que l'on retrouve dans les céréales, dans l'orge et l'avoine en particulier. Les fibres de β - glucane peuvent également être extraites de la paroi cellulaire des plantes vertes, et de certaines algues et champignons (maitaké et shiitake). Dans le cadre de l'invention, les fibres de β - glucane sont des fibres intactes, c'est-à-dire que les fibres ne sont pas préalablement modifiées par un traitement chimique ou enzymatique, tel que par l'emploi de cellulases exogènes. Les fibres intactes de β - glucane ont le plus souvent une masse moléculaire de l'ordre de 220 000 Da ± 10 000 Da (masse moléculaire mesurée par chromatographie par perméation de gel).

La source en fibres de β - glucane est le plus souvent un extrait d'avoine ou d'orge, enrichi en lesdites fibres. De tels extraits sont disponibles dans le commerce. En particulier, la société Cargill propose un extrait purifié de β - glucane d'orge, avec une teneur en β - glucane supérieure à 70% (sous dénomination commerciale Barliv™).

Les maltodextrines sont des produits obtenus par hydrolyse de l'amidon. Dans le cadre de la présente invention, on utilise des maltodextrines ayant un degré d'hydrolyse (DE) inférieur à 18. En effet, les inventeurs ont constaté que seules les maltodextrines faiblement hydrolysées permettaient de réduire la viscosité de la solution aqueuse et d'éviter sa gélification.

Le degré d'hydrolyse caractérise le degré de polymérisation des maltodextrines : des maltodextrines de faible DE sont des maltodextrines peu hydrolysées, encore appelées maltodextrines à longue chaîne.

Dans le cadre de la présente invention, les maltodextrines ont avantageusement un DE inférieur à 10, encore plus avantageusement inférieur à 6.

Dans le cadre de la présente invention, la gomme de guar au moins partiellement hydrolysée a avantageusement un poids moléculaire compris entre 10 000 et 100 000 Da, plus avantageusement entre 10 000 et 50 000 Da, encore plus avantageusement entre 10 000 et 30 000 Da. En particulier, la gomme de guar au moins partiellement hydrolysée peut avoir une masse moléculaire en poids d'environ 20 000 Da.

Cette gomme de guar au moins partiellement hydrolysée est avantageusement obtenue suite à une hydrolyse enzymatique de gomme de guar.

Les inulines sont des oligosaccharides naturels (d'origine végétale principalement) contenant des unités fructose. On les retrouve principalement dans la chicorée (source principale des produits actuellement sur le marché), les oignons, les poireaux, l'ail, les bananes, les asperges et les artichauts.

Dans le cadre de la présente invention, les inulines ont avantageusement un poids moléculaire compris entre 1 000 et 5 000 Da.

Le terme fructo-oligosaccharides (FOS) fait référence à des oligosaccharides à petite chaîne comprenant des unités D-fructose et D-glucose, contenant classiquement de 3 à 5 unités monosaccharidiques.

Dans le cadre de la présente invention, les fructo-oligosaccharides ont avantageusement un poids moléculaire compris entre 500 et 1 000 Da.

Tous ces dépresseurs de viscosité sont des composés solubles avec un faible pouvoir texturant. En particulier, on constatera que leur poids moléculaire est avantageusement inférieur à 50 000 Da, plus avantageusement inférieur à 30 000 Da. Ces dépresseurs de viscosité présentent tous la capacité de former une phase continue possédant une viscosité raisonnable (limitée). Les fibres de β - glucane vont former une phase dispersée qui s'intégrera dans la phase continue.

Dans le cadre de la présente invention, l'expression « faible pouvoir texturant » signifie que les dépresseurs de viscosité présentent une viscosité à 10% inférieure à 1 000 mPa.s.

A titre de dépresseur de viscosité, on préférera la gomme de guar au moins partiellement hydrolysée qui ne pose pas de problème de tolérance et qui est peu calorique, contrairement aux maltodextrines qui ont un apport calorifique pouvant, pour certaines applications, être considéré comme étant trop important ou aux inulines qui peuvent poser des problèmes de tolérance digestive.

La teneur en fibres de β - glucane dans la solution aqueuse selon l'invention varie de 3% à 12% en poids, par rapport au poids total de ladite solution. Des teneurs inférieures à 3% en poids ne présenteraient que peu d'intérêt sur le plan alimentaire. Par ailleurs, une solution aqueuse comprenant plus de 12% en poids de fibres de β - glucane est difficilement réalisable.

La teneur en fibres de β - glucane dans la solution aqueuse selon l'invention varie avantageusement de 3% à 10%, plus avantageusement de 3% à 8%, encore plus avantageusement de 3% à 7% en poids, et encore plus avantageusement de 3 à 6,5% en poids, par rapport au poids total de ladite solution. Des résultats très satisfaisant peuvent toutefois également être obtenus avec des teneurs en fibres de β - glucane élevées, c'est-à-dire de l'ordre de 8 à 10% en poids, par rapport au poids total de ladite solution (cf. exemples 4 et 6).

La teneur en dépresseur de viscosité dans la solution aqueuse selon l'invention varie de 5% à 30% en poids, par rapport au poids total de ladite solution.

La teneur en dépresseur de viscosité dépend d'une part de la teneur en fibres de β - glucane et d'autre part de la cinétique de la vitesse de refroidissement, dans le procédé d'obtention.

Plus la teneur en fibres de β - glucane dans la solution est importante, plus il faut introduire de dépresseur de viscosité. En parallèle, plus la cinétique de refroidissement, lors du procédé d'obtention, est lente, moins on a besoin d'introduire de dépresseur de viscosité.

Une méthode de détermination de la teneur minimale en dépresseur de viscosité à ajouter est expliquée dans l'exemple 3.

Selon une variante avantageuse de l'invention, la solution aqueuse comprend 3% à 5% en poids desdites fibres de β-glucane et 5% à 20%, plus avantageusement 8% à 15%, dudit dépresseur de viscosité, par rapport au poids total de la solution.

Selon une autre variante avantageuse de l'invention, la solution aqueuse comprend 5% à 8% en poids desdites fibres de β-glucane et 7% à 20%, plus avantageusement 12% à 15%, en poids dudit dépresseur de viscosité, par rapport au poids total de la solution.

Selon encore une autre variante avantageuse de l'invention, la solution aqueuse comprend 8% à 12%, avantageusement 8% à 10%, en poids desdites fibres de β - glucane et 10% à 30% dudit dépresseur de viscosité, par rapport au poids total de la solution.

Les teneurs en dépresseur de viscosité indiquées correspondent aux teneurs nécessaires pour que les composés macromoléculaires jouent leur rôle de dépresseur de viscosité. On pourrait en introduire des quantités plus importantes. Ainsi, on pourrait ajouter des teneurs supérieures à 30% en poids de gomme de guar au moins partiellement hydrolysée qui jouerait alors d'une part son rôle de dépresseur de viscosité et qui, d'autre part, apporterait des fibres au produit fini.

La solution aqueuse selon l'invention peut en outre comprendre un concentré de jus de fruits, de la purée de fruits concentrée et/ou des morceaux de fruits.

L'invention a également pour objet un procédé pour préparer une solution aqueuse thermisée selon l'invention.

Ce procédé comprend une étape de refroidissement lent d'une dispersion thermisée comprenant
- de l'eau,
- au moins un dépresseur de viscosité, et
- des fibres de β-glucanes,
sous cisaillement, jusqu'à une température comprise entre 4°C et 30°C.

En particulier, ce procédé comprend les étapes successives suivantes :
a. Dispersion du dépresseur de viscosité et des fibres de β - glucane dans l'eau ;
b. Chauffage de la dispersion obtenue suite à l'étape précédente jusqu'à température de chambrage et maintien de cette dispersion à ladite température de chambrage ;
c. Refroidissement lent de la dispersion obtenue suite à l'étape b), sous cisaillement, jusqu'à une température comprise entre 4°C et 30°C.

Les inventeurs ont constaté que, outre l'ajout d'un dépresseur de viscosité, l'étape de refroidissement lent sous cisaillement était essentielle pour introduire des fibres de β-glucane dans une solution aqueuse en évitant à la fois une augmentation trop importante de la viscosité et une prise en masse (gélification). En effet, en cas de refroidissement brutal, on constate une gélification de la solution obtenue. Ceci est également observé dans le cas d'un refroidissement statique (c'est à dire sans cisaillement).

Des dispersions ayant des valeurs de viscosité supérieures à 10 000 mPa.s posent des problèmes significatifs de pompabilité. Dans le cadre de la présente invention, une dispersion semi-fluide est une dispersion qui a une viscosité (mesurée à 10°C) inférieure à 10 000 mPa.s.

Le refroidissement est avantageusement réalisé à une vitesse maximale de 2°C/min.

Pour une teneur fixée en dépresseur de viscosité, plus la cinétique de refroidissement est lente, plus la teneur en fibres de β - glucane qui peut être introduite est importante.

Sur le plan économique, on conçoit difficilement un refroidissement s'étendant sur plus d'une journée. Le refroidissement est donc avantageusement réalisé à une vitesse comprise entre 0,15°C/min et 1°C/min.

Lors du refroidissement, la vitesse de cisaillement est généralement comprise entre 10 s⁻¹ et 800 s⁻¹, avantageusement entre 50 s⁻¹ et 500 s⁻¹, plus avantageusement entre 50 et 300 s⁻¹. Il semblerait que la vitesse de cisaillement n'ait qu'un faible impact sur la viscosité et la prise en masse de la solution aqueuse semi-fluide obtenue. Un cisaillement lors du refroidissement est cependant absolument nécessaire.

Les conditions de thermisation correspondent à celles classiquement utilisées dans le domaine alimentaire. Ainsi, la température de chambrage est avantageusement comprise entre 80°C et 95°C. Par ailleurs, la durée de chambrage varie avantageusement de 2 minutes à 20 minutes.

Le procédé peut comprendre, à la suite de l'étape a) et préalablement à l'étape b), une étape d'ajout à la dispersion obtenue d'un concentré de jus de fruits, de purée de fruits concentrée, de morceaux de fruits et/ou de sucre.

Lors de l'étape c), la solution aqueuse est refroidie jusqu'à sa température d'utilisation ou de stockage / conservation. Dans le domaine alimentaire, une température de 10°C est une température de conservation classique.

Ce procédé ne nécessite pas de conditions opératoires difficiles à maîtriser, telles qu'un travail sous pression. Les températures mises en oeuvre sont tout à fait classiques. Il est donc facilement industrialisable.

L'invention a également pour objet un produit alimentaire semi-fluide comprenant la solution aqueuse pasteurisée selon l'invention.

Un produit alimentaire semi-fluide est un produit alimentaire ayant une activité de l'eau (AW) supérieure à 0,90. L'activité de l'eau est le ratio de la pression de vapeur d'eau pour un produit sur la pression de vapeur de l'eau pure à une même température.

Ce produit alimentaire comprend avantageusement 0,5 à 5% en poids de fibres de β-glucane, par rapport à son poids total.

Ce produit alimentaire est avantageusement choisi dans le groupe constitué par les produits à base de soja, les produits à base de fruits et/ou de légumes, les fourrages pour produits céréaliers, et les produits laitiers.

Les produits laitiers sont en particuliers des produits laitiers fermentés.

Par "produits laitiers fermentés", on entend plus particulièrement des produits laitiers fermentés prêts à la consommation humaine, c'est-à-dire des aliments laitiers fermentés. Dans la présente demande, sont plus particulièrement visés les laits fermentés et yoghourts. Lesdits aliments laitiers fermentés peuvent alternativement être des fromages blancs ou des petits-suisses.

On donne aux termes "laits fermentés" et " yoghourts " leurs significations usuelles dans le domaine de l'industrie laitière, c'est à dire des produits qui sont destinés à la consommation humaine, et qui sont issus de la fermentation lactique acidifiante d'un substrat laitier. Ces produits peuvent contenir des ingrédients secondaires tels que fruits, végétaux, sucre, etc. On peut par exemple se reporter au Décret français n°88-1203 du 30 décembre 1988 relatif aux laits fermentés et au yaourt ou yoghourt, publié au Journal Officiel de la République Française du 31 décembre 1988.

On peut également se reporter au "Codex Alimentarius " (préparé par la Commission du Codex Alimentarius sous l'égide de la FAO et de l'OMS, et publié par la Division Information de la FAO, disponible en ligne sur http://www.codexalimentarius.net; cf plus particulièrement le volume 12 du Codex Alimentarius "Normes Codex pour le lait et les produits laitiers ", et la norme " CODEX STANA-1 1(a)-1975").

Le terme " lait fermenté " est ainsi réservé dans la présente demande au produit laitier préparé avec un substrat laitier qui a subi un traitement au moins équivalent à la pasteurisation, ensemencé avec des microorganismes appartenant à l'espèce ou aux espèces caractéristiques de chaque produit. Un " lait fermenté " n'a subi aucun traitement permettant de soustraire un élément constitutif du substrat laitier mis en oeuvre, et notamment n'a pas subi un égouttage du coagulum. La coagulation des " laits fermentés " ne doit pas être obtenue par d'autres moyens que ceux qui résultent de l'activité des microorganismes utilisés.

Le terme " yoghourt " est quant à lui réservé au lait fermenté obtenu, selon les usages locaux et constants, par le développement des bactéries lactiques thermophiles spécifiques dites *Lactobacillus bulgaricus* et *Streptococcus thermophilus,* qui doivent se retrouver vivantes dans le produit fini, à raison d'au moins 10 millions de bactéries par gramme rapportées à la partie lactée.

Dans certains pays, la réglementation autorise l'ajout d'autres bactéries lactiques dans la production de yoghourt, et notamment l'utilisation additionnelle de souches de *Bifidobacterium* et/ou de *Lactobacillus acidophilus* et/ou de *Lactobacillus casei.*

Ces souches lactiques additionnelles sont destinées à conférer au produit fini diverses propriétés, telles que la propriété de favoriser l'équilibre de la flore intestinale, ou de moduler le système immunitaire.

Dans la pratique, le terme "lait fermenté" est donc généralement utilisé pour désigner les laits fermentés autres que yoghourts, et peut prendre, selon les pays, le nom de " Kefir ", Kumiss ", Lassi ", Dahi ", Leben ", Filmjôlk ", Villi ", " Acidophilus milk " par exemple.

La quantité d'acide lactique libre contenue dans le substrat laitier fermenté ne doit pas être inférieure à 0,6 g pour 100 g lors de la vente au consommateur, et la teneur en matière protéique apportée à la partie lactée ne doit pas être inférieure à celle d'un lait normal.

La dénomination " fromage blanc " ou " petit-suisse " est, dans la présente demande, réservée à un fromage non affiné, non salé, qui a subi une fermentation par des bactéries lactiques uniquement (pas d'autre fermentation que la fermentation lactique).

La teneur en matière sèche des fromages blancs peut être abaissée jusqu'à 15 g ou 10 g pour 100 g de fromage blanc, selon que leur teneur en matière grasse est 25% supérieure à 20 g, ou au plus égale à 20 g, pour 100 g de fromage blanc, après complète dessiccation. La teneur en matière sèche d'un fromage blanc est comprise entre 13 et 20 %. La teneur en matière sèche d'un petit-suisse quant à elle n'est pas inférieure à 23 g pour 100 g de petit-suisse. Elle est généralement comprise entre 25 et 30 %. Les fromages blancs et petits suisses sont généralement regroupés sous la dénomination « fromages frais » utilisée de manière classique dans le domaine technique de la présente invention.

L'invention a également pour objet la solution aqueuse thermisée semi-fluide selon l'invention, ou le produit alimentaire la comprenant, en tant qu'aliment fonctionnel.

Le terme « aliment fonctionnel » est ainsi réservé dans la présente demande à des aliments ayant la capacité d'influencer de façon bénéfique des fonctions du corps de manière pour améliorer la sensation de bien-être et la santé et réduire le risque d'apparition de maladies (définition ILSI : Concepts of functional foods by M. Ashwell - 2002).

En particulier, ledit aliment fonctionnel a les visées thérapeutiques suivantes : prévention des maladies cardiovasculaires, du diabète, de l'obésité, et prévention ou traitement du surpoids.

Ledit aliment fonctionnel peut particulièrement être utilisé dans le cadre d'un régime alimentaire à visée amincissante ou de gestion du poids.

Ladite solution ou ledit produit alimentaire peut être utilisé dans une alimentation à visée diététique, en particulier pour réduire le taux de cholestérol sanguin ou pour diminuer le taux d'insuline post-prandial.

Les exemples qui suivent illustrent l'invention.

### Exemple 1 : Préparation de solutions semi-fluides à 5% de β - glucane :

On prépare des solutions aqueuses comprenant 5% en poids de fibres de β - glucane (7,15% de Barliv™) et :
- Aucun dépresseur de viscosité (exemple comparatif) ;
- 10% en poids de maltodextrine DE6 ;
- 10 % en poids de gomme de guar hydrolysée (PHGG).

La maltodextrine utilisée est la référence Glucide® 6 (Roquette). Pour le guar hydrolysé, la référence utilisée est le Sunfiber® R (Taiyo Kagaku).

Les essais ont été réalisés au laboratoire ou dans une cellule viscosimétrique, dans les conditions suivantes :
- dispersion des poudres (fibres de β - glucane, maltodextrine, PHGG) dans l'eau froide,
- chauffage du mix sous agitation,
- chambrage de quelques minutes à une température de 90°C ou 95°C,
- refroidissement statique ou dynamique (sous cisaillement) jusqu'à 10°C, et mesure de la viscosité à une vitesse de cisaillement de 64 s⁻¹,
- stockage à 10°C de la solution obtenue, et observation de la prise éventuelle de gel.

De manière générale, le β - glucane se disperse facilement sous agitation dans l'eau froide. On obtient alors une solution faiblement visqueuse, que le β-glucane soit mis en solution seul ou avec un dépresseur de viscosité. Une augmentation de la viscosité est observée vers 60°C lors du chauffage (viscosité de 0,15 Pa.s à 60 °C et à 150 s-1), correspondant à l'hydratation du β - glucane.

Les résultats observés pour différents mélanges sont résumés dans le tableau 1, où la durée du refroidissement a été fixée à 120 minutes et le cisaillement à 150 s⁻¹ :

**Tableau 1**

| | **Refroidissement** | **Viscosité 10°C J0 (mPa.s)** | **Prise de gel 10°C J+1** |
|---|---|---|---|
| 5% β - glucane | statique | 5 900 | oui |
| | dynamique | 5 900 | oui |
| 5% β - glucane + 10% maltodextrine DE 6 | statique | Gel | oui |
| | dynamique | 5450 | non |
| 5% β - glucane + 10% PHGG | statique | 2900 | oui |
| | dynamique | 1200 | non |

On constate que l'addition de 10% de PHGG permet de réduire significativement la viscosité développée après traitement thermique. Cependant, cette addition n'empêche pas la gélification du système, qui se produit au bout de quelques heures lors d'un refroidissement statique.

Seule la combinaison : ajout d'un dépresseur de viscosité + refroidissement dynamique permet de générer des solutions à 5% de β - glucane possédant une viscosité réduite, qui ne gélifient pas lors d'un stockage à 10°C.

Par ailleurs, la durée de refroidissement est un paramètre qui conditionne la viscosité finale et la gélification du système.

Un refroidissement rapide (quelques dizaines de secondes à quelques minutes) ne permet pas d'obtenir le résultat souhaité, alors qu'un refroidissement plus lent (quelques dizaines de minutes à quelques heures) s'avère plus favorable.

Le tableau 2, où les mesures ont été réalisées sur le système 5% β - glucane + 10% PHGG, à une vitesse de cisaillement de 150 s⁻¹ lors du refroidissement, résume ces observations.

**Tableau 2**

| **Durée de refroidissement (min.)** | **Viscosité 10°C J0 (mPa.s)** | **Prise de gel 10°C J+1** |
|---|---|---|
| 1 | 2500 | Oui |
| 5 | 2460 | Oui |
| 30 | 2400 | Oui |
| 60 | 1 800 | Non |
| 120 | 1 200 | Non |

Lorsque la durée de refroidissement est suffisamment importante (au-delà d'une heure), aucune prise de gel n'est observée sur 28 jours lors d'une conservation de la solution à 10°C.

### Exemple 2 : Préparation de solutions semi-fluides à 5,7% de β - glucane :

On prépare des solutions aqueuses comprenant 5,7% en poids de fibres de β - glucane (8,14% de Barliv™) et 10 %, 12,5% et 15% en poids de gomme de guar hydrolysée (PHGG ; Sunfiber^{®} R (Taiyo Kagaku)).

Les ingrédients sont mélangés ensemble puis dispersés sous agitation à l'aide d'un mobile de type défloculeuse à 800 tours par minute pendant une heure au moins. Le mélange est traité thermiquement dans une cuve double enveloppe à 95°C pendant 10 minutes. Le refroidissement s'effectue sous cisaillement (env. 100s⁻¹) et en deux heures afin d'empêcher une forte reprise de gel.

Les solutions suivantes sont obtenues :

**Tableau 3**

| | Barliv™ | PHGG | Eau | Masse blanche/Solution |
|---|---|---|---|---|
| Solution A | 8,14% (5,7% de beta-glucane) | 10% | 81,86% | 60/40 |
| Solution B | | 12,5% | 73,3% | |
| Solution C | | 15% | 68,3% | |

Aucune des solutions réalisées ne reprend en texture. Les solutions sont stables dans le temps, aucune séparation de phase n'est visible après 21 jours de conservation à 10°C.

Chaque solution est incorporée dans une masse blanche de type velouté (ratio massique masse blanche/solution : 60/40), pour indication la viscosité de ladite masse blanche est comprise entre 1100 et 1200 mPa.s. Le tableau 4 ci-dessous montre le suivi des viscosités des produits finis.

**Tableau 4 : viscosité à 64 s⁻¹, 10 s de yoghourts brassés, au cours du stockage à froid (10°C)**

| | Viscosité à 64 s⁻¹ (mPas) / Aspect | | | |
|---|---|---|---|---|
| | J + 1 | J + 7 | J + 14 | J + 28 |
| Mélange solution B | 1178 | 1141 | 1159 | 1153 |
| | Non gélifié | Non gélifié | Non gélifié | Non gélifié |
| Mélange solution C | 1580 | 1575 | 1588 | 1596 |
| | Non gélifié | Non gélifié | Non gélifié | Non gélifié |

Les viscosités des produits finis sont très intéressantes car une fois mélangés, les solutions ne viscosifient pas le milieu de façon importante comme le fait la gomme de guar seule. Les produits finis sont stables à J+28, aucune synérèse n'est visible.

### Exemple 3 : Obtention d'une préparation à base de jus de fruits contenant 6.4 % de β - glucane :

Le β-glucane et le guar hydrolysé (Sunfiber^{®} R, Taiyo Kagaku) sont mis en solution par dispersion à froid dans de l'eau à laquelle est ajouté ensuite le jus de fruit concentré. Le sucre en poudre et le sirop de glucose sont ensuite ajoutés, puis la solution est ensuite acidifiée avant d'appliquer les conditions de traitement thermique décrites à l'exemple 1. Des systèmes d'agitation classiques sont suffisants pour disperser les ingrédients.

Des préparations à base de jus de fruits contenant 9% de Barliv™, soit 6,4% de β-glucane ont été préparées.

Les résultats observés sont résumés dans le tableau 5, où la durée du refroidissement a été fixée à 120 minutes et le cisaillement à 150 s⁻¹.

**Tableau 5**

| | **Refroidissement** | **Viscosité 10°C J0 (mPa.s)** | **Prise de gel 10°C J+1** |
|---|---|---|---|
| 6.4 % β - glucane + 10% PHGG | dynamique | 4600 | non |

### Exemple 4 : Détermination des concentrations optimales de β-glucane et de gomme de guar partiellement hydrolysée permettant d'obtenir une solution selon l'invention

Une quantité déterminée de gomme de guar partiellement hydrolysée (PHGG : Sunfiber R^{®} - Taiyo Kagaku - Fiderstadt, Germany) et de fibres de β-glucane (Barliv™ - Cargill - Minneapolis, MN, USA) sont dispersées dans de l'eau. Cette dispersion est ensuite chauffée jusqu'à 95°C et maintenue à cette température. La dispersion est enfin refroidie lentement (pendant 120 minutes), sous cisaillement (150 s⁻¹), jusqu'à 10°C.

La viscosité (mesurée à l'aide d'un rhéomètre PHYSICA UDS 200 - Anton Paar) de la solution obtenue est mesurée juste après fabrication (J0) et un jour après cette fabrication (J+1).

L'aspect de la solution obtenue est ensuite évalué à J+1. Le point de comparaison pour cette évaluation est l'aspect d'un produit n'ayant pas subi le cisaillement mais ayant subi le même traitement thermique.

Les résultats obtenus sont donnés dans le tableau 6 suivant :

**Tableau 6**

| **Concentration (%w/w)** | | **Viscosité (mPa.s à 64 s⁻¹)** | **Aspect** |
|---|---|---|---|
| **β-glucane** | **PHGG** | **J0** | **J+1** |
| 3 | 6 | 1870 | Non gélifié |
| 3,5 | 5 | 2750 | gélifié |
| 5 | 5 | 7520 | gélifié |
| 5 | 7,5 | 5750 | gel très faible |
| 8 | 15 | 7530 | Non gélifié |

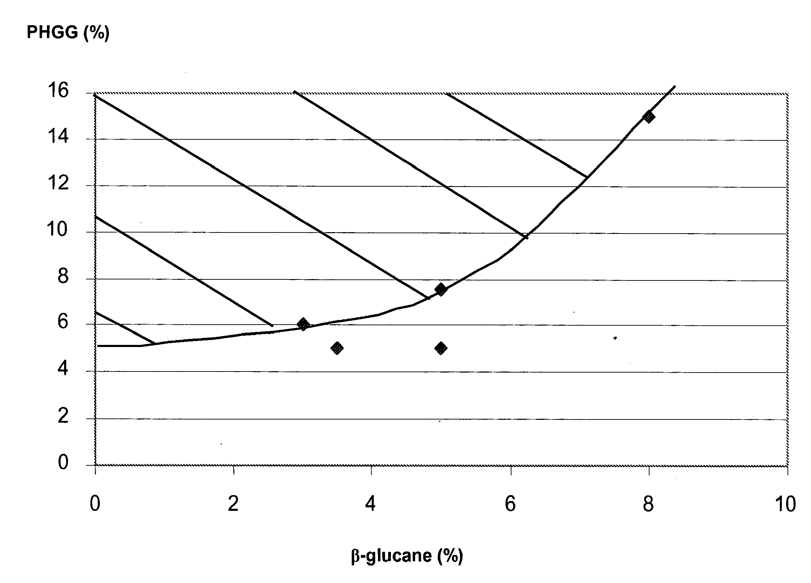

Ils sont par ailleurs reportés sur la figure 1, sur laquelle la teneur (en % massique) en PHGG est reportée en ordonnés et à la teneur en β - glucane (% massique) est reportée en abscisse.

La zone hachurée, au dessus de la courbe, correspond à la zone dans laquelle la dispersion ne gélifie pas (zone de « non gel ») alors que la zone en dessous de la courbe correspond à une zone de gélification (zone de « gel »).

La courbe obtenue donne la teneur minimale en PHGG nécessaire par rapport à la teneur en β - glucane désirée, pour une cinétique de refroidissement.

Les teneurs minimales en PHGG pour d'autres cinétiques de refroidissement peuvent facilement être déterminées en reproduisant l'enseignement de cet exemple 3 adapté à la cinétique choisie.

### Exemple 5 : Préparation de produits laitiers fermentés contenant du β - glucane :

### Produits de type yoghourt brassé :

Un produit proche d'un yoghourt brassé aux fruits texturé a pu être obtenu par mélange 50/50 d'un yoghourt brassé nature (d'une viscosité de 1050 mPa.s à 10 °C) et d'une solution à 6,4% de β - glucane telle que décrite ci-dessus.

L'opération de mélange ne présente pas de difficulté particulière et peut être réalisée en utilisant des mélangeurs classiques.

Ce produit présente une viscosité de 1800 mPa.s. à 10°C, il possède des propriétés organoleptiques acceptables et il est stable lors d'une conservation à 10°C pendant 28 jours.

125 g de ce produit contiennent une dose de 4 g de β - glucane.

### Produits de type yoghourt brassé :

Un produit proche d'un yoghourt brassé aux fruits plus fluide a pu être obtenu par mélange 81/19 d'un yoghourt brassé nature (de viscosité de 1050 mPa.s à 10 °C) et d'une solution à 6,4% de β - glucane telle que décrite ci-dessus.

L'opération de mélange ne présente pas de difficulté particulière et peut être réalisée en utilisant des mélangeurs classiques.

Ce produit présente une viscosité de 1070 mPa.s. à 10°C, il possède des propriétés organoleptiques acceptables et il est stable lors d'une conservation à 10°C pendant 28 jours.

125 g de ce produit contiennent une dose de 1,5 g de β - glucane.

### Boissons lactées fermentées :

Une boisson lactée fermentée aux fruits a pu être obtenue par mélange de 88 % d'un lait fermenté nature à boire (de viscosité 30 mPa.s) et de 12 % d'une préparation à base de jus de fruits contenant 6,4 % de β - glucane telle que décrite ci-dessus.

L'opération de mélange ne présente pas de difficulté particulière et peut être réalisée en utilisant des mélangeurs classiques.

Ce produit présente une viscosité de 280 mPa.s. à 10°C, il possède des propriétés organoleptiques acceptables et il est stable lors d'une conservation à 10°C pendant 28 jours.

100 g de cette boisson contiennent une dose de 0,75 g de β - glucane.

### Exemple 6 : Préparation de solutions semi-fluides à 9,31% de β - glucane :

On prépare des solutions aqueuses comprenant 9,31% en poids de fibres de β-glucane (8,14% de Barliv™) 20% en poids de gomme de guar hydrolysée (PHGG ; Sunfiber^{®} R (Taiyo Kagaku)).

Les ingrédients sont mélangés ensemble puis dispersés sous agitation à l'aide d'un mobile de type défloculeuse à 800 tours par minute pendant une heure au moins. Le mélange est traité thermiquement dans une cuve double enveloppe à 95°C pendant 10 minutes. Le refroidissement s'effectue sous cisaillement (env. 100s⁻¹) et en deux heures afin d'empêcher une forte reprise de gel.

Les solutions suivantes sont obtenues :

**Tableau 7**

| | Barliv™ | PHGG | Eau | Masse blanche/Solution |
|---|---|---|---|---|
| Solution D | 13,3% (9,31% de beta-glucane) | 20% | 66,7% | 70/30 |

Cette solution ne reprend pas en texture. Elle est stable dans le temps, aucune séparation de phase n'est visible après 21 jours de conservation à 10°C.

Cette solution est incorporée dans une masse blanche de type velouté (ratio massique masse blanche/solution : 70/30), pour indication la viscosité de ladite masse blanche est comprise entre 1100 et 1200 mPa.s. Le tableau 8 ci-dessous montre le suivi des viscosités des produits finis.

**Tableau 8 : viscosité à 64 s⁻¹, 10 s de yoghourts brassés, au cours du stockage à froid (10°C)**

| | Viscosité à 64 s⁻¹ (mPas) | | | |
|---|---|---|---|---|
| | J + 1 | J + 7 | J + 14 | J + 28 |
| Mélange solution D | 1604 | 1571 | 1604 | 1598 |
| | Non gélifié | Non gélifié | Non gélifié | Non gélifié |

Les viscosités des produits finis sont très intéressantes car une fois mélangés, la solution ne viscosifie pas le milieu de façon importante comme le fait la gomme de guar seule. Les produits finis sont stables à J+28, aucune synérèse n'est visible. Cette solution D permet en outre l'utilisation d'une teneur importante (70% en poids) de masse blanche.

## Revendications

1. Solution aqueuse thermisée semi-fluide comprenant des fibres de β - glucane, **caractérisée en ce qu'**elle comprend
a. 3% à 12% en poids, par rapport au poids total de la solution, desdites fibres de β - glucane, et
b. une quantité suffisante, comprise entre 5% et 30% en poids, par rapport au poids total de la solution, d'au moins un dépresseur de viscosité choisi dans le groupe constitué par les maltodextrines ayant un DE maximal de 18, la gomme de guar au moins partiellement hydrolysée, l'inuline et les fructo oligosaccharides.

2. Solution selon la revendication 1, **caractérisée en ce qu'**elle comprend 3% à 10%, avantageusement 3% à 8%, encore plus avantageusement 3% à 7% en poids desdites fibres de β - glucane, par rapport au poids total de la solution.

3. Solution selon l'une quelconque des revendications précédentes, **caractérisée en ce qu'**elle comprend 3% à 5% en poids desdites fibres de β - glucane et 5% à 20% dudit dépresseur de viscosité, par rapport au poids total de la solution, avantageusement 5% à 8% en poids desdites fibres de β - glucane et 7% à 20% dudit dépresseur de viscosité, par rapport au poids total de la solution.

4. Solution selon l'une quelconque des revendications précédentes, **caractérisée en ce qu'**elle comprend 8% à 12%, avantageusement 8% à 10%, en poids desdites fibres de β - glucane et 10% à 30% dudit dépresseur de viscosité, par rapport au poids total de la solution.

5. Solution selon l'une quelconque des revendications précédentes, **caractérisé en ce que** le dépresseur de viscosité est la gomme de guar au moins partiellement hydrolysée.

6. Solution selon l'une quelconque des revendications précédentes, **caractérisée en ce qu'**elle comprend également un concentré de jus de fruits, de la purée de fruits concentrée et/ou des morceaux de fruits.

7. Procédé pour préparer une solution aqueuse thermisée selon l'une quelconque des revendications 1 à 6, **caractérisé en ce qu'**il comprend une étape de refroidissement lent d'une dispersion thermisée comprenant de l'eau, au moins un dépresseur de viscosité et des fibres de β - glucane, sous cisaillement, jusqu'à une température comprise entre 4°C et 30°C.

8. Procédé selon la revendication 7, comprenant les étapes successives suivantes :
a. Dispersion du dépresseur de viscosité et des fibres de β - glucane dans l'eau ;
b. Chauffage de la dispersion obtenue suite à l'étape précédente jusqu'à température de chambrage avantageusement comprise entre 80°C et 95°C et maintien de cette dispersion à ladite température de chambrage ;
c. Refroidissement lent de la dispersion obtenue suite à l'étape b), sous cisaillement, jusqu'à une température comprise entre 4°C et 30°C.

9. Procédé selon l'une quelconque des revendications 7 ou 8, **caractérisé en ce que** le refroidissement est réalisé à une vitesse maximale de 2°C/min, avantageusement à une vitesse comprise entre 0,15°C/min et 1°C/min.

10. Procédé selon l'une quelconque des revendications 7 à 9, **caractérisé en ce que** la vitesse de cisaillement, lors du refroidissement, est comprise entre 10 et 800 s⁻¹, avantageusement entre 50 et 300 s⁻¹.

11. Procédé selon l'une quelconque des revendications 7 à 10, **caractérisé en ce que** la durée de chambrage varie de 2 minutes à 20 minutes.

12. Procédé selon l'une quelconque des revendications 8 à 11, comprenant, à la suite de l'étape a) et préalablement à l'étape b), une étape d'ajout à la dispersion obtenue, d'un concentré de jus de fruits, de purée de fruits concentrée, de morceaux de fruits et/ou de sucre.

13. Utilisation d'une solution aqueuse thermisée selon l'une quelconque des revendications 1 à 6, pour préparer un produit alimentaire semi-fluide, le produit alimentaire comprenant avantageusement 0,5 à 5% en poids de fibres de β - glucane, par rapport à son poids total.

14. Utilisation selon la revendication 13, **caractérisée en ce que** le produit alimentaire est choisi dans le groupe constitué par les produits à base de soja, les produits à base de fruits et/ou de légumes, les fourrages pour produits céréaliers, et les produits laitiers.

15. Solution selon l'une quelconque des revendications 1 à 6, sous forme d'aliment fonctionnel.

16. Solution selon l'une quelconque des revendications 1 à 6, pour son utilisation dans la prévention des maladies cardiovasculaires, du diabète, de l'obésité et la prévention et/ou le traitement du surpoids et/ou pour réduire le taux de cholestérol sanguin et/ou pour diminuer le taux d'insuline post-prandial.

## Patentansprüche

1. Thermisierte halbflüssige wässrige Lösung, die β-Glucan-Fasern umfasst, **dadurch gekennzeichnet, dass** sie Folgendes umfasst:
a. 3 Gew.-% bis 12 Gew.-% der β-Glucan-Fasern, bezogen auf das Gesamtgewicht der Lösung, und
b. eine ausreichende Menge mindestens eines Viskositätveeminderungsmittels zwischen 5 Gew.-% und 30 Gew.-%, bezogen auf das Gesamtgewicht der Lösung, wobei das die Viskosität senkende Mittel aus der Gruppe bestehend aus Maltodextrinen mit einem maximalen DE von 18, zumindest partiell hydrolysiertem Guargummi, Inulin und Fruktooligosacchariden ausgewählt ist.

2. Lösung nach Anspruch 1, **dadurch gekennzeichnet, dass** sie 3 Gew.-% bis 10 Gew.-%, vorteilhafterweise 3 Gew.-% bis 8 Gew.-%, noch vorteilhafterweise 3 Gew.-% bis 7 Gew.-% der β-Glucan-Fasern, bezogen auf das Gesamtgewicht der Lösung, umfasst.

3. Lösung nach einem der vorhergehenden Ansprüche, **dadurch gekennzeichnet, dass** sie 3 Gew.-% bis 5 Gew.-% der β-Glucan-Fasern und 5 Gew.-% bis 20 Gew.-% des Viskositätveeminderungsmittels, bezogen auf das Gesamtgewicht der Lösung, vorteilhafterweise 5 Gew.-% bis 8 Gew.-% der β-Glucan-Fasern und 7 Gew.-% bis 20 Gew.-% des Viskositätveeminderungsmittels, bezogen auf das Gesamtgewicht der Lösung, umfasst.

4. Lösung nach einem der vorhergehenden Ansprüche, **dadurch gekennzeichnet, dass** sie 8 Gew.-% bis 12 Gew.-%, vorteilhafterweise 8 Gew.-% bis 10 Gew.-% der β-Glucan-Fasern und 10 Gew.-% bis 30 Gew.-% des Viskositätveeminderungsmittels, bezogen auf das Gesamtgewicht der Lösung, umfasst.

5. Lösung nach einem der vorhergehenden Ansprüche, **dadurch gekennzeichnet, dass** das Viskositätveeminderungsmittels zumindest partiell hydrolysiertes Guargummi ist.

6. Lösung nach einem der vorhergehenden Ansprüche, **dadurch gekennzeichnet, dass** sie außerdem ein Fruchtsaftkonzentrat, konzentriertes Fruchtpüree und/oder Fruchtstücke umfasst.

7. Verfahren zur Herstellung einer thermisierten wässrigen Lösung nach einem der Ansprüche 1 bis 6, **dadurch gekennzeichnet, dass** das Verfahren einen Schritt des langsamen Abkühlens einer thermisierten Dispersion, die Wasser, mindestens ein Viskositätveeminderungsmittel und β-Glucan-Fasern umfasst, unter Scheren auf eine Temperatur zwischen 4 °C und 30 °C umfasst.

8. Verfahren nach Anspruch 7, das die folgenden sukzessiven Schritte umfasst:
a. Dispergieren des Viskositätveeminderungsmittels und der β-Glucan-Fasern in dem Wasser;
b. Erhitzen der nach dem vorherigen Schritt erhaltenen Dispersion auf eine Haltetemperatur, die vorteilhafterweise zwischen 80 °C und 95 °C liegt, und Halten dieser Dispersion auf dieser Haltetemperatur;
c. langsames Abkühlen der nach Schritt b) erhaltenen Dispersion unter Scheren auf eine Temperatur zwischen 4 °C und 30 °C umfasst.

9. Verfahren nach einem der Ansprüche 7 oder 8, **dadurch gekennzeichnet, dass** das Abkühlen mit einer maximalen Geschwindigkeit von 2 °C/min, vorteilhafterweise mit einer Geschwindigkeit zwischen 0,15 °C/min und 1 °C/min durchgeführt wird.

10. Verfahren nach einem der Ansprüche 7 bis 9, **dadurch gekennzeichnet, dass** die Schergeschwindigkeit während des Abkühlens zwischen 10 und 800 s⁻¹, vorteilhafterweise zwischen 50 und 300 s⁻¹ liegt.

11. Verfahren nach einem der Ansprüche 7 bis 10, **dadurch gekennzeichnet, dass** die Haltedauer von 2 Minuten bis 20 Minuten variiert.

12. Verfahren nach einem der Ansprüche 8 bis 11, das nach dem Schritt a) und vor dem Schritt b) einen Schritt des Zugebens eines Fruchtsaftkonzentrats, eines konzentrierten Fruchtpürees, von Fruchtstücken und/oder von Zucker zu der erhaltenen Dispersion umfasst.

13. Verwendung einer thermisierten wässrigen Lösung nach einem der Ansprüche 1 bis 6 zum Herstellen eines halbflüssigen Lebensmittelprodukts, wobei das Lebensmittelprodukt vorteilhafterweise 0,5 bis 5 Gew.-% β-Glucan-Fasern, bezogen auf sein Gesamtgewicht, umfasst.

14. Verwendung nach Anspruch 13, **dadurch gekennzeichnet, dass** das Lebensmittelprodukt aus der Gruppe bestehend aus Produkten auf Sojabasis, Produkten auf Frucht- und/oder Gemüsebasis, Füllstoffe für Getreideprodukte und Milchprodukten ausgewählt ist.

15. Lösung nach einem der Ansprüche 1 bis 6 in der Form eines funktionellen Lebensmittels.

16. Lösung nach einem der Ansprüche 1 bis 6 für ihre Verwendung bei der Verhinderung von Herz-Kreislauf-Erkrankungen, Diabetes und Adipositas und der Verhinderung und/oder Behandlung von Übergewicht und/oder zum Senken des Blutcholesterinspiegels und/oder zum Senken des postprandialen Insulinspiegels.

## Claims

1. Semi-fluid thermised aqueous solution containing ß-glucan fibres, **characterized in that** it comprises:
a. 3% to 12% by weight, relative to the total weight of the solution, of said ß-glucan fibres, and
b. a sufficient quantity, of between 5% and 30% by weight, relative to the total weight of the solution, of at least one viscosity depressant chosen from the group consisting of maltodextrins having a maximum DE of 18, at least partly hydrolysed guar gum, inulin and fructo-oligosaccharides.

2. Solution according to claim 1, **characterized in that** it comprises 3% to 10% , advantageously 3% to 8%, further advantageously 3% to 7% by weight of said ß-glucan fibres, relative to the total weight of the solution.

3. Solution according to anyone of the preceding claims, **characterized in that** it comprises 3% to 5% by weight of said ß-glucan fibres and 5% to 20% by weight of said viscosity depressant, relative to the total weight of the solution, advantageously 5% to 8% by weight of said ß-glucan fibres and 7% to 20% of said viscosity depressant, relative to the total weight of the solution.

4. Solution according to anyone of the preceding claims, **characterized in that** it comprises 8% to 12%, advantageously 8% to 10% by weight of said ß-glucan fibres, and 10% to 30% of said viscosity depressant, relative to the total weight of the solution.

5. Solution according to anyone of the preceding claims, **characterized in that** the viscosity depressant is at least partly hydrolysed guar gum.

6. Solution according to anyone of the preceding claims **characterized in that** it also comprises a fruit juice concentrate, a concentrated fruit purée and/or fruit pieces.

7. Process to prepare a thermised aqueous solution according to anyone of claims 1 to 6, **characterized in that** it comprises a slow cooling step of a thermised dispersion containing water, at least one viscosity depressant and ß-glucan fibres, under shear, down to a temperature of between 4°C and 30°C.

8. Process according to claim 7, comprising the following successive steps:
a. Dispersion of the viscosity depressant and ß-glucan fibres in water;
b. Heating the dispersion obtained after the preceding step up to a holding temperature advantageously between 80°C and 95°C and holding said dispersion at said holding temperature;
c. Slow cooling of the dispersion obtained after step b), under shear, down to a temperature of between 4°C and 30°C.

9. Process according to anyone of claim 7 or 8, **characterized in that** cooling is conducted at a maximum rate of 2°C/min, advantageously at a rate of between 0.15°C/min and 1°C/min.

10. Process according to anyone of claims 7 to 9, **characterized in that** the shear rate, during cooling, lies between 10 s⁻¹ and 800 s⁻¹, advantageously between 50 s⁻¹ and 300 s⁻¹.

11. Process according to anyone of claims 7 to 10, **characterized in that** the holding time varies between 2 minutes and 20 minutes.

12. Process according to anyone of claims 8 to 11 which, after step a) and prior to step b), comprises a step to add to the dispersion obtained a concentrate of fruit juice, concentrated fruit purée, fruit pieces and/or sugar.

13. Use of a thermised aqueous solution according to anyone of claims 1 to 6, to prepare a semi-fluid food product, the food product containing advantageously 0.5% to 5% by weight ß-glucan fibres, relative to its total weight.

14. Use according to claim 13, **characterized in that** the food product is chosen from the group consisting of soy-based products, fruit and/or vegetable-based products, fillers for cereal products, and dairy products.

15. Solution according to anyone of claims 1 to 6, in the form of functional food.

16. Solution according to anyone of claims 1 to 6, for use in the prevention of cardiovascular disease, diabetes, obesity and the prevention and/or treatment of overweight and/or to reduce blood cholesterol levels and/or to reduce postprandial insulin levels.
